# DEMANDE DE BREVET EUROPEEN

(11) **EP 2 810 698 A1**
(43) Date de publication de la demande: **10.12.2014**
(21) Numéro de dépôt: 14169954.6
(22) Date de dépôt: 27.05.2014
(51) Int. Cl.: A62B 9/00, F16J 15/02, G01F 15/18, G01L 19/06, A61M 16/00

(54) **Système de mesure de pression de gaz pour appareil de ventilation de patient**

(30) Priorité: 06.06.2013 FR 1355204
(71) Demandeur: Air Liquide Medical Systems, 92160 Antony (FR)
(72) Inventeur: Germani, Damien, 75015 Paris (FR); Guiducci, Hadrien, 75018 Paris (FR)
(74) Mandataire: Pittis, Olivier

(57) **Abrégé**

L'invention porte sur un système de mesure de pression de gaz (6, 16) comprenant au moins un circuit de gaz (10) et au moins un capteur de pression (6) agencé de manière à pouvoir mesurer la pression du gaz dans au moins une partie du circuit de gaz (10), ledit au moins un capteur de pression (6) étant protégé par une membrane de protection (1) perméable au gaz, agencée entre ledit au moins un capteur de pression (6) et le circuit de gaz (10). Il comporte en outre une pièce intermédiaire souple (8) formée d'un matériau élastiquement déformable agencée entre ledit au moins un capteur de pression (6) et la membrane de protection (1), ladite pièce intermédiaire souple (8) comprenant un passage interne (8a) mettant en communication fluidique la membrane de protection (1) et ledit au moins un capteur de pression (6). Appareil de ventilation (20) de patient comprenant un circuit de gaz (10) apte à acheminer du gaz entre une source de gaz (23) et un patient, ainsi qu'un tel système de mesure de pression de gaz (6, 16).

## Description

L'invention concerne un système de mesure de pression de gaz comprenant au moins un capteur de pression et une membrane protectrice, dans lequel une pièce intermédiaire souple permet d'assurer l'étanchéité du capteur et de sa prise de pression tout en les protégeant de l'humidité, en particulier l'humidité provenant du circuit de gaz patient d'un appareil de ventilation médicale d'un patient comprenant un circuit de gaz ventilé équipé d'un tel système.

Dans certains appareils médicaux de mesure de débit de gaz, l'air allant au patient est humide. Or, les capteurs de pression sont très sensibles à l'humidité.

Par ailleurs, les capteurs ont une géométrie variable, c'est-à-dire des formes et des dimensions pouvant être sensiblement différentes d'une version de capteur à l'autre.

Dès lors, le problème qui se pose est de pouvoir réaliser une étanchéité du capteur de pression d'un circuit patient et d'obtenir une protection de celui-ci contre l'humidité et ce, quel que soit le capteur utilisé, c'est-à-dire quelle que soit sa géométrie.

En d'autres termes, il existe un besoin pour un système permettant d'amener du gaz, tel de l'air, ou une pression gazeuse au capteur tout en empêchant les fuites vers l'extérieur et par ailleurs apte à protéger le capteur de l'humidité, voire même à assurer une barrière antibactérienne.

Parmi les solutions existantes, on connait certains systèmes utilisant plusieurs pièces usinées ou moulées rigides associées à des joints souples et/ou de la colle pour maintenir la membrane protégeant le capteur.

Or, ces solutions engendrent des inconvénients ou conduisant à d'autres problèmes comme des difficultés de montage, une impossibilité de démontage dans le cas où de la colle est utilisée, un risque d'oubli de joints lors du montage, des coûts importants liés au nombre de pièces et à leurs manipulations, un risque de détérioration de la membrane qui est parfois très fragile par l'une ou l'autres des pièces rigides utilisées en entrainant alors des fuites inopinées, un risque de détérioration du joint lors du montage, une zone d'étanchéité faible, voire insuffisante...

D'autres solutions incluant des capteurs possédant leur propre connexion existent.

Dans ce cas, il est nécessaire de raccorder ensuite les capteurs et aux orifices de prise de mesure avec moyens de connexion, tels des tuyaux, des olives de connexion...

Toutefois, ces types de capteurs sont bien plus imposants. Ainsi, leur taille peut être 10 à 20 fois supérieure à celle des capteurs classiques, par exemple de l'ordre de 1 mm pour un capteur classique contre 10 à 20 mm pour les capteurs à connectivité propre.

Ce type de capteurs pose donc des problèmes d'insertion et d'utilisation de ce type de capteurs dans certains appareils médicaux, en particulier ceux de taille réduite, et engendre donc une augmentation notable de leur encombrement.

En d'autres termes, les systèmes de mesure de pression de gaz existants ne sont pas totalement sans poser certains problèmes et engendrer certains inconvénients.

Dès lors, le problème qui se pose est de proposer un système de mesure de pression de gaz pour appareil médical qui mette en oeuvre un nombre de pièces réduit, soit de coûts faibles, présente moins de risque de fuites, soit de montage facile et rapide, conduise à moins de risques d'oubli lors du montage, soit démontable en production et maintenance, engendre moins de risques de fuites avec détérioration des joints et/ou d'abimer la ou les membranes fragiles...

La solution est alors un système de mesure de pression de gaz comprenant au moins un circuit de gaz et au moins un capteur de pression agencé de manière à pouvoir mesurer la pression du gaz dans au moins une partie du circuit de gaz, ledit au moins un capteur de pression étant protégé par une membrane de protection perméable au gaz, agencée entre ledit au moins un capteur de pression et le circuit de gaz, caractérisé en ce qu'il comporte en outre une pièce intermédiaire souple formée d'un matériau élastiquement déformable agencée entre ledit au moins un capteur de pression et la membrane de protection, ladite pièce intermédiaire souple comprenant un passage interne mettant en communication fluidique la membrane de protection et ledit au moins un capteur de pression.

Selon le cas, le système de mesure de pression de gaz de l'invention peut comprendre l'une ou plusieurs des caractéristiques techniques suivantes :
- il comprend deux capteurs de pression, chaque capteurs de pression étant protégé par une membrane de protection perméable au gaz et une pièce intermédiaire souple formée d'un matériau élastiquement déformable étant agencée entre chaque capteur de pression et la membrane de protection.
- il comprend deux capteurs de pression dont les prises de pression sont reliées au circuit de gaz de part et d'autre d'un moyen de création de perte de charge au sein dudit circuit de gaz, en particulier une restriction de passage.
- la pièce intermédiaire souple a une forme de révolution.
- la pièce intermédiaire souple comprenant un passage interne qui peut être central ou excentré.
- la pièce intermédiaire souple comprend un évidement interne amont situé du côté de la membrane de protection, et un logement interne aval situé du côté dudit au moins un capteur de pression, et l'évidement interne amont et le logement interne aval étant reliés fluidiquement l'un à l'autre par le passage interne.
- l'évidement interne amont et le logement interne aval ont un diamètre (ou une dimension) de passage de gaz supérieur à celui du passage interne.
- la pièce intermédiaire souple comprend un logement interne aval dans lequel est positionné ledit au moins un capteur de pression.
- de manière alternative, la pièce intermédiaire souple comprend un logement interne aval débouchant autour de l'orifice de prise de pression porté par ledit au moins un capteur de pression.
- la pièce intermédiaire souple est formé d'un matériau polymère ou élastomère, de préférence un matériau de type élastomère thermoplastique TPE ou silicone.
- la pièce intermédiaire souple comprend un rebord aval délimitant le logement interne aval, ledit rebord aval venant prendre appui de manière étanche, autour dudit au moins un capteur de pression, sur la paroi portant ledit au moins un capteur de pression ou sur ledit au moins un capteur lui-même, de préférence autour de l'orifice de prise de pression dudit (ou des) capteur.
- la pièce intermédiaire souple comprend une bordure amont délimitant l'évidement interne amont et venant appuyer sur la membrane de protection.
- un boitier 7 comprenant un orifice d'entrée de gaz (7a) situé en regard de l'évidement amont (8b), permet de maintenir la pièce intermédiaire souple (8) au contact de la membrane de protection (1) et en position autour du capteur de pression (6).
- le boitier comprend un couvercle.
- le boitier comprend un premier embout portant un orifice d'entrée de gaz et un second embout portant un orifice de sortie de gaz, lesdits orifice d'entrée de gaz et orifice de sortie de gaz étant reliés par un passage de gaz interne, de préférence ledit passage de gaz interne forme une partie du circuit de gaz.
- la pièce intermédiaire souple comprend une paroi périphérique externe coopérant avec la paroi interne du boitier de manière à assurer une étanchéité fluidique entre elles.
- le capteur de pression est porté par une carte électronique formant tout ou partie de la paroi sur laquelle la pièce intermédiaire souple vient prendre appui de manière étanche, via son rebord aval, autour dudit capteur de pression.
- la carte électronique portant le ou les capteurs est fixée dans le boitier, de préférence par vissage, de manière à maintenir la membrane de protection et la pièce

intermédiaire souple solidaires l'une de l'autre et assurer en outre une étanchéité entre la pièce intermédiaire souple et la paroi portant le capteur de pression.
- la pièce intermédiaire souple comprend une paroi périphérique externe munie d'expansions de paroi vers l'extérieur, c'est-à-dire ayant une forme obtenue par révolution, telle une lèvre ou analogue.

L'invention concerne en outre un appareil de ventilation de patient comprenant un circuit de gaz apte à acheminer du gaz entre une source de gaz et un patient, caractérisé en ce qu'il comporte en outre un système de mesure de pression de gaz selon l'invention, notamment tel que décrit ci-dessus.

De préférence le système de mesure de pression de gaz est agencé sur le circuit de gaz, de préférence au sein d'un boitier venant se raccorder fluidique audit circuit de gaz ou à une ligne de dérivation dudit circuit de gaz.

L'invention va maintenant être mieux comprise grâce à la description détaillée suivante faite en références aux Figures annexées parmi lesquelles :
- la Figure 1 représente un premier mode de réalisation du système de mesure de pression selon l'invention pour appareil de ventilation artificielle,
- la Figure 2 représente une vue d'un premier côté (face inférieure) de la pièce intermédiaire souple du système de mesure de pression de la Figure 1,
- la Figure 3 représente une vue d'un second côté (face supérieure) opposé au premier côté de la pièce intermédiaire souple du système de mesure de pression de la Figure 1,
- la Figure 4 est une vue schématique d'un mode de réalisation d'un appareil de ventilation de patient équipé d'un système de mesure de pression selon la Figure 1 intégré dans un boitier, et
- la Figure 5 est un schéma d'un mode de réalisation particulier de la paroi périphérique externe de la pièce intermédiaire souple des Figures 1 à 3.
- les Figures 6A et 6B représentent un mode de réalisation du système de mesure de la Figure 1 intégré à un boitier venant se raccorder au circuit de gaz d'un appareil comme illustré en Figure 4, et
- la Figure 7 représente un deuxième mode de réalisation de la pièce souple d'un système de mesure de pression selon l'invention.

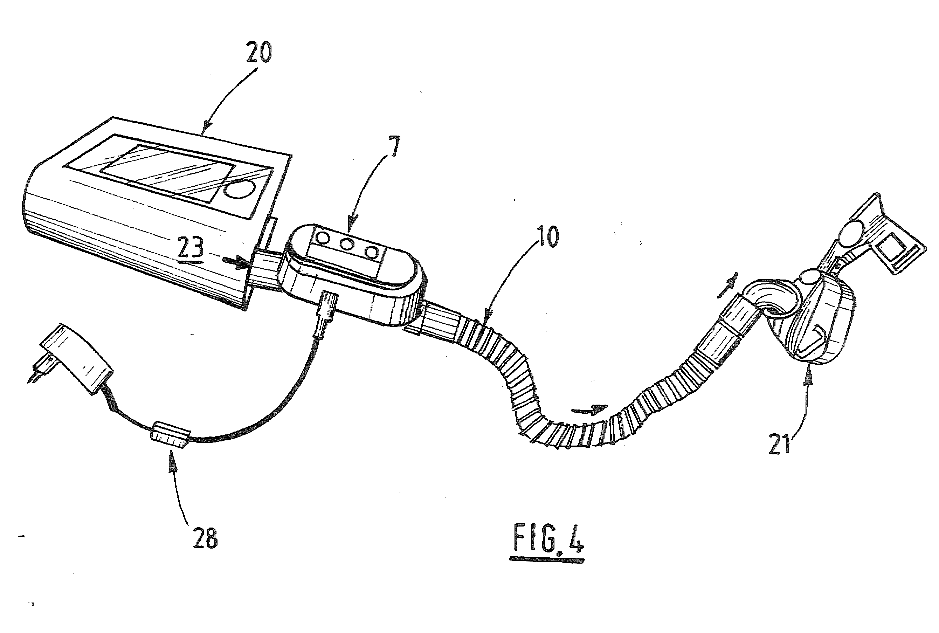

La Figure 4 représente un schéma d'un mode de réalisation d'un appareil 20 de ventilation artificielle, c'est-à-dire un ventilateur médical, tel par exemple le ventilateur médical MONNAL™ T50 commercialisé par la Demanderesse, comprenant un circuit de gaz 10, appelé circuit patient, lequel comprend une branche respiratoire unique, encore appelée branche inspiratoire, alimentée fluidiquement en gaz par une source de gaz 23 (non visible), telle une turbine ou une micro-soufflante, située dans l'appareil 20 et qui est apte à et conçu pour délivrer un débit de gaz sous pression, tel de l'air, c'est-à-dire à une pression supérieure à 1 atm, i.e. pression atmosphérique. Le sens du flux de gaz est schématisé par les flèches le long de la branche inspiratoire.

Le circuit patient 10 permet de convoyer le gaz issu de la source de gaz 23 jusqu'à une interface patient 21, tel un masque, qui permet de délivrer le débit de gaz à un patient (non représenté).

Afin de pouvoir déterminer la pression du gaz dans au moins une partie du circuit patient 10, on utilise classiquement un système de mesure de pression de gaz 6, 16 comprenant un ou plusieurs capteur(s) de pression 6 et au moins une carte électronique 16 reliée au(x)dit(s) capteurs de pression 6.

La ou les prises de pression 24 du ou desdits capteurs 6 de pression communiquent fluidiquement avec le circuit patient 10 via un ou plusieurs orifices 7a.

On peut par exemple utiliser deux capteurs 6 de pression agencés en série avec les membranes 1 et les pièces déformables 8 agencées dans des logements dédiés 34, 36.

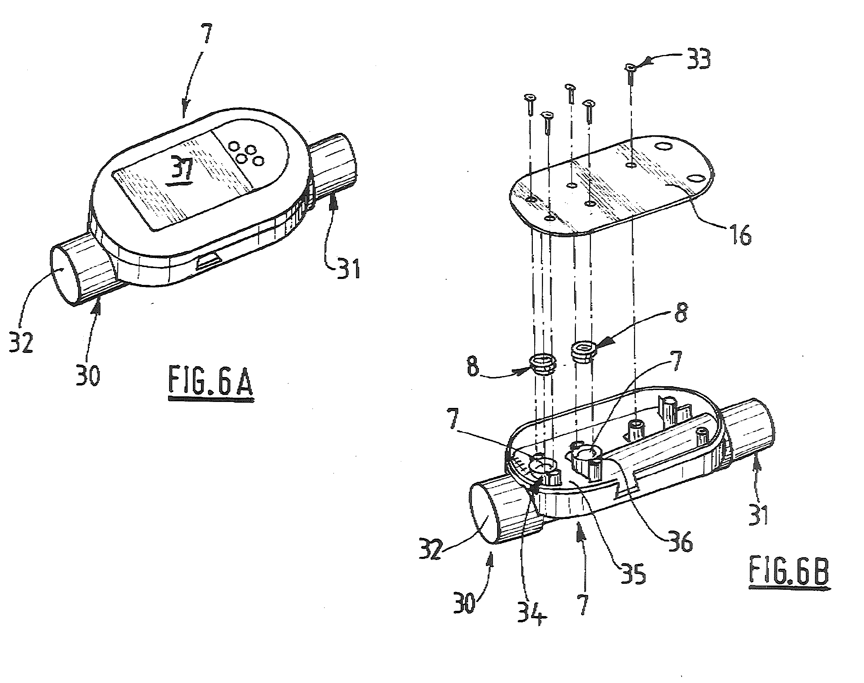

Les capteurs 6 ont leurs prises de pression 24 séparées l'une de l'autre par un moyen de création de pertes de charge, telle une restriction de passage 35 par exemple. Un tel agencement permet notamment d'opérer des mesures de pression différentielle. Une telle architecture est illustrée en Figure 6B, comme expliqué ci-dessous.

Afin de simplifier la compréhension, le mode de réalisation décrit ci-après ne comporte qu'un seul capteur de pression 6 mais l'invention vise également à s'appliquer à des systèmes de mesure de pression à plusieurs capteurs 6, notamment à deux capteurs.

Selon le mode de réalisation choisi, le (ou les) capteur de pression 6 peut être agencé de manière à pouvoir mesurer la pression du gaz circulant dans le circuit de gaz 10:
- soit dans le flux de gaz, c'est-à-dire comme sur la Figure 4 en étant intégré dans un boitier 7 raccordé au circuit patient 10, en sortie de ventilateur 20 par exemple, et alimenté en courant électrique par un cordon d'alimentation 28 classique,
- soit dans la paroi du circuit 10, c'est-à-dire en étant intégré à ladite paroi par exemple,
- soit dans une ligne de dérivation amenant la pression ou un débit de fluide au capteur 6.

Afin de protéger le ou chaque capteur de pression 6 des contaminants, tel que vapeur d'eau (humidité), poussières, microorganismes...., susceptibles de se trouver dans le flux de gaz et/ou dans le circuit patient 10, le ou chaque capteur 6 est protégé par une membrane de protection 1 perméable au gaz agencée en amont dudit capteur 6, dans un logement ou une ligne de dérivation permettant de faire une prise de pression entre le circuit de gaz 10 et le capteur de pression 6.

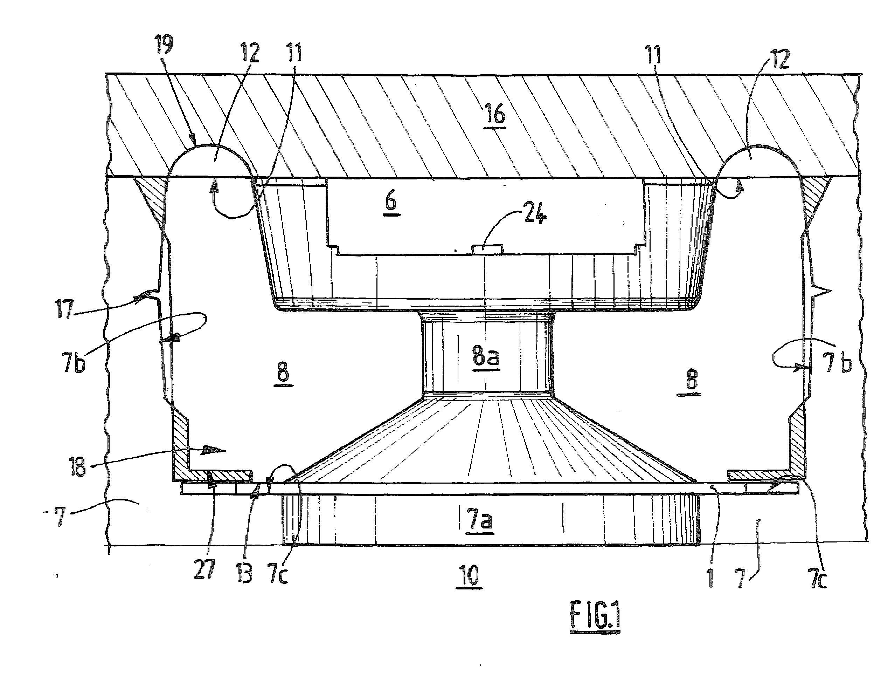

Selon la présente invention, comme illustré en Figure 1, le système de mesure de pression selon l'invention comporte en outre une pièce intermédiaire souple 8 formée d'un matériau élastiquement déformable agencée entre le capteur de pression 6 et la membrane de protection 1.

Cette pièce intermédiaire souple 8 comprend un passage interne 8 a, tel un passage central, mettant en communication fluidique la membrane de protection 1 et le capteur de pression 6 de manière permettre à la pression de gaz régnant dans le circuit patient 10 de s'exercer successivement au travers de la membrane 1, dans le passage interne 8a et jusqu'au capteur de pression 6 où ce dernier peut la mesurer, via la prise de pression 24 du capteur 6 qui est située sur ou dans le capteur 6. Ici, la prise de pression 24 est un orifice permettant à la pression de rentrer à l'intérieur du capteur 6.

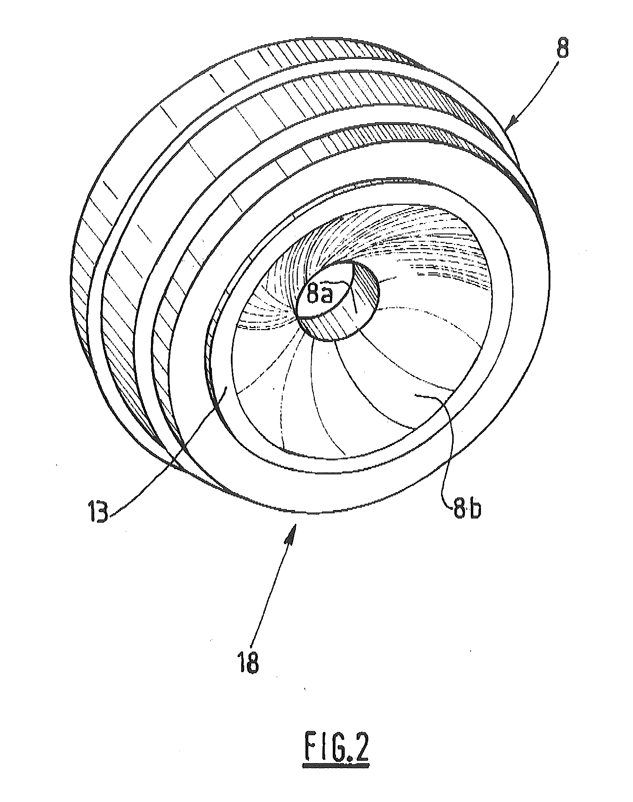

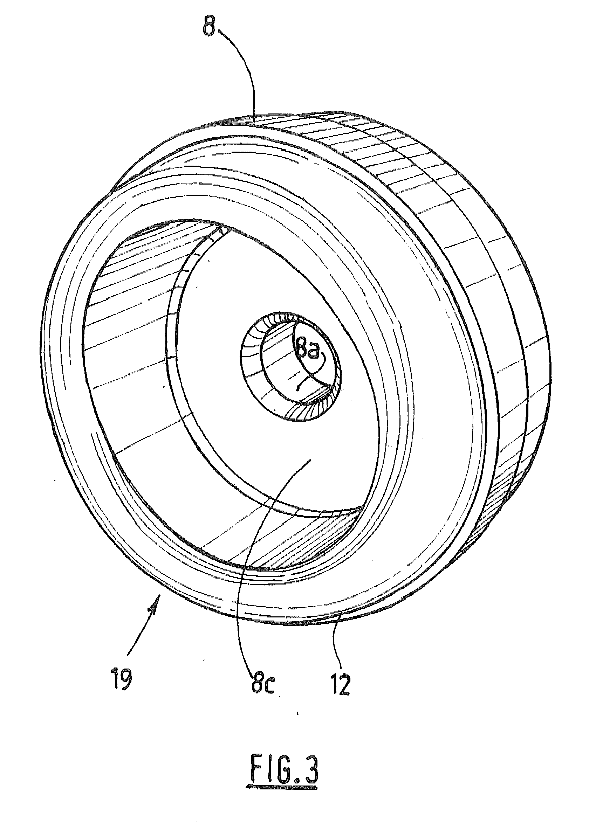

Comme visible sur les Figures 2 et 3, la pièce intermédiaire souple 8 est une pièce de révolution, donc de forme générale tridimensionnelle. Ici, elle comporte une périphérie circulaire mais cette pièce 8 peut avoir d'autres formes, par exemple polygonale, notamment cubique, hexagonale ou octogonale.

Elle comprend en outre un évidement interne amont 8b situé du côté de la membrane de protection 1, et un logement interne aval 8c situé du côté du capteur de pression 6, qui sont reliés fluidiquement l'un à l'autre par le passage interne 8a.

Ici, l'évidement interne amont 8b a une forme tronconique, c'est-à-dire que son diamètre interne diminue progressivement en direction du passage interne 8a.

Cependant, l'évidement interne amont 8b peut avoir toute autre forme dès lors que le fluide est amené au capteur 6 au travers du passage 8a, qui lui forme un goulot d'étranglement, et du logement interne aval 8c.

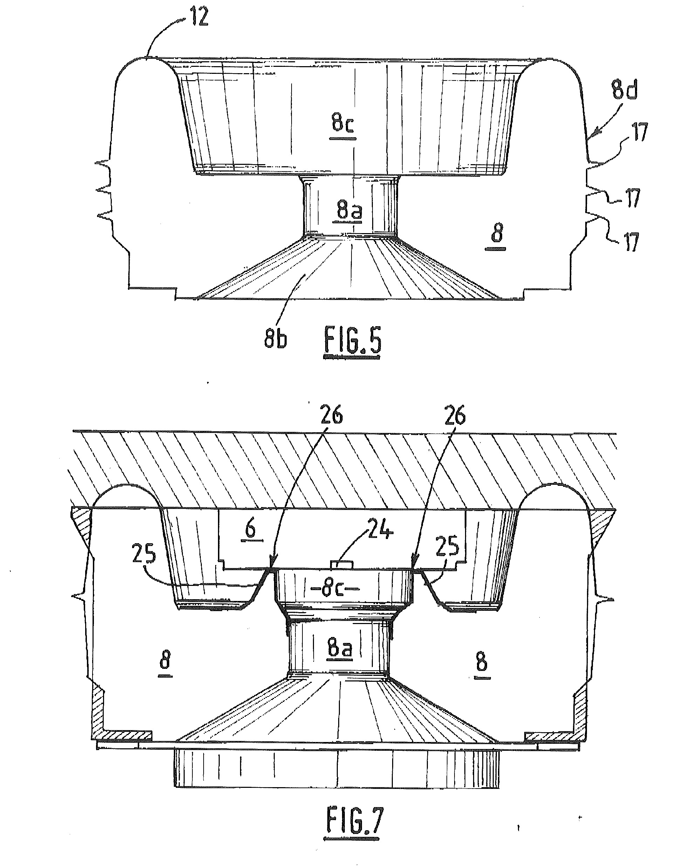

D'ailleurs, concernant le logement interne aval 8c, il est à souligner que l'étanchéité peut aussi être fait directement sur le capteur 6 en modifiant la forme dudit logement interne aval 8c de manière à réduire le volume de ce logement interne aval 8c tel qu'il formerait une chambre ou un passage dans la continuité du passage interne 8a dont une expansion de paroi 25 se projetterait vers l'arrière et viendrait prendre appui directement (en 26) autour de l'orifice 24 de prise de pression du capteur 6 et y créer une étanchéité gazeuse, comme illustré par le mode de réalisation particulier de la Figure 7.

Dans le mode de réalisation de la Figure 1, la pièce intermédiaire souple 8 vient se positionner autour du capteur de pression 6 de manière à ce que son logement interne aval 8c vient former une chambre protectrice englobant ledit capteur de pression 6.

Afin d'assurer sa fonction notamment d'étanchéité fluidique, la pièce intermédiaire souple 8 est formé d'un matériau polymère ou élastomère, de préférence un matériau de type élastomère thermoplastique couramment appelé TPE ou silicone.

En fait, dans le mode de réalisation illustré en Figures 1 et 3, la pièce intermédiaire souple 8 comprend, du côté de sa face arrière 19, un rebord aval 12 délimitant le logement aval interne 8c et venant prendre appui de manière étanche autour du capteur de pression 6, c'est-à-dire sur la paroi 11 portant le capteur de pression 6, en particulier sur une carte électronique 16 pouvant constituer cette paroi 11 dans certains modes de réalisation.

Ceci permet d'assurer une étanchéité fluidique entre la pièce intermédiaire souple 8 et la paroi 11 portant le capteur de pression 6, étant donné que la pièce 8 élastique se déforme légèrement en venant s'écraser contre la paroi 11 (Fig. 1) ou contre le capteur 6 lui-même (Fig. 7), selon le mode de réalisation considéré.

Comme représenté en Figure 2, sur sa face opposée, c'est-à-dire la face avant 18, la pièce intermédiaire souple 8 porte une bordure amont 13 délimitant l'évidement amont 8b et venant appuyer sur la membrane de protection 1 et plus précisément sur le pourtour de la face arrière de la membrane 1 de manière à la maintenir en position contre le fond 7c du boitier 7, comme expliqué ci-dessous en rapport avec la Figure 6B notamment.

Ceci permet aussi d'assurer une étanchéité fluidique entre la pièce intermédiaire souple 8 et la membrane 1. En particulier, comme illustré en Figure 1, on prévoit ici un palier 27 notamment entre la pièce 8 et la membrane 1 pour créer une sorte de pincement de la membrane 1 ; toutefois, on pourrait avoir prévoir une autre forme, comme une surface plate ou une petite lèvre de révolution.

En fait, l'étanchéité fluidique du système de mesure est assurée par compression et déformation du matériau de la pièce intermédiaire souple 8 qui est pris en sandwich et légèrement écrasé et déformé élastiquement entre le fond 7c du boitier 7 et la paroi 11 portant le capteur de pression 6, comme visisble sur la Figure 1.

Cette déformation et d'éventuelles formes de révolution comme les ailettes 17 schématisées en Figure 5, assurent l'étanchéité fluidique entre la paroi périphérique externe 8d de la pièce 8 et la surface interne 7c du boitier 7, en particulier la surface interne 7c d'un ou plusieurs logements dédiés 34, 36 aménagés dans le boitier 7 et destinés à recevoir la ou les membranes 1 et la ou les pièces déformables 8, comme illustré en Figure 6B.

En effet, dans un mode de réalisation particulier, la pièce intermédiaire souple 8 comprend une paroi périphérique externe 8d munie d'une ou plusieurs expansions de paroi 17 se projetant vers l'extérieur, de préférence plusieurs expansions de paroi 17 de révolution agencées en parallèle les unes des autres, comme schématisé en Figure 5. Ces expansions de paroi 17 permettent d'améliorer l'étanchéité en diminuant l'écrasement radial nécessaire.

Par ailleurs, la paroi 16 portant le (ou les) capteur 6, en particulier une carte électronique, vient de fixer par vissage de manière à maintenir la membrane de protection 1 et la pièce intermédiaire souple 8 solidaires l'une de l'autre et assurer en outre l'étanchéité.

En fait, la pièce intermédiaire souple 8 entrant dans le cadre de la présente invention permet de plaquer la membrane 1 au fond 7c du boitier 7 et/ou d'un logement dédié 34, 36, sans l'abimer, et assurer ainsi l'étanchéité entre la membrane 1 et la pièce intermédiaire souple 8 elle-même, tout en laissant la possibilité au fluide de passer au travers de la membrane 1 et d'être ainsi filtré.

Du côté opposé, la pièce intermédiaire souple 8 permet de faire l'étanchéité au niveau du capteur 6, c'est-à-dire dans les régions de paroi 11, 16 entourant le (ou les) capteur 6, c'est-à-dire le capteur 6 lui-même, la paroi-support ou carte électronique 16...

Une fois mise en place, l'ensemble assure aussi une étanchéité avec l'extérieur et permet d'apporter la pression, i.e. le gaz filtré par la membrane 1, tel de l'air, au ou à chaque capteur de pression 6.

En outre, le rétrécissement constituant tout ou partie du passage interne 8a de la pièce intermédiaire souple 8 permet de garantir en outre une bonne rigidité de l'ensemble qui fait que l'ensemble de la pièce intermédiaire souple 8 forme un joint apte à maintenir une force de contact efficace sur les zones d'étanchéités stuées sur les côtés.

Les Figures 6A et 6B représentent un mode de réalisation du système de mesure de la Figure 1 intégré à un boitier 7 venant se raccorder au circuit 10 de gaz d'un appareil ventilateur comme celui de la Figure 4. Sur la Figure 6A, le boitier est vu fermé, alors que la Figure 6B montre une vue éclatée dudit boitier 7.

Plus précisément, ce boitier 7 comprend un passage 32 de gaz interne formant une partie du circuit de gaz 10 reliant une entrée de gaz portée par un embout d'entrée 30 à une sortie de gaz portée par un embout de sortie 31, entre lesquelles circule le gaz respiratoire délivré par la source de gaz 23 de l'appareil 20 de ventilation et alimentant un patient via l'interface 21 alimentée par un conduit souple formant une autre partie du circuit de gaz 10, comme illustré en Figure 1.

En fait, le boitier 7 vient se raccorder entre l'appareil 20 et le conduit souple 10 comme illustré sur la Figure 1, au niveau des embouts d'entrée et sortie.

Comme on le voit sur la Figure 6B, le boitier 7 comporte ici deux capteurs de pression (non visibles) agencés en série et portés par la carte électronique 16 du côté de la face 11 par exemple de la Figure 6B, ladite carte électronique 16 étant fixée par exemple par vissage 33.

Les pièces déformables 8 viennent quant à elle s'insérer dans des logements dédiés 34, 36.

Lesdits capteurs 6 ont par ailleurs leurs prises de pression 24 espacées l'une de l'autre en étant séparées par un moyen de création de pertes de charge, par exemple une restriction de passage 35 placée sur le passage interne 32 de gaz, ce qui permet de réaliser des mesures différentielles de pression.

Le boitier 7 est ensuite refermé par un couvercle 37 comme illustré en Figure 6A, qui protège les composants internes et évite les entrées de poussière et autres contaminants.

Il est à souligner que les mesures de pression faites par le(s) capteur(s) de pression 6 sont traitées de manière classique par la carte électronique 16, en particulier par un ou des microprocesseurs ou analogue.

Dans tous les cas, les avantages d'un système de mesure de pression d'un appareil selon l'invention sont notamment :
- une simplicité de montage sans omission de pièce possible,
- un temps de montage très réduit,
- un risque de fuite diminué car du fait de zones d'étanchéité en nombre réduit, i.e. 3 zones seulement,
- une fiabilité accrue puisque la membrane ne risque pas d'être détériorée par une pièce coupante...

Le système de mesure de pression et l'appareil de ventilation équipé d'un tel système de mesure de pression selon l'invention peuvent être utilisés pour alimenter en gaz respiratoire les patients souffrant d'insuffisances respiratoires, par exemple ceux souffrant de pathologies respiratoires de type par exemple SDRA, SAS (apnée du sommeil) et/ou ceux devant observer un traitement de type oxygénothérapie ou analogue.

## Revendications

1. Système de mesure de pression de gaz (6, 16) comprenant au moins un circuit de gaz (10) et au moins un capteur de pression (6) agencé de manière à pouvoir mesurer la pression du gaz dans au moins une partie du circuit de gaz (10), ledit au moins un capteur de pression (6) étant protégé par une membrane de protection (1) perméable au gaz, agencée entre ledit au moins un capteur de pression (6) et le circuit de gaz (10), **caractérisé en ce qu'**il comporte en outre une pièce intermédiaire souple (8) formée d'un matériau élastiquement déformable agencée entre ledit au moins un capteur de pression (6) et la membrane de protection (1), ladite pièce intermédiaire souple (8) comprenant un passage interne (8a) mettant en communication fluidique la membrane de protection (1) et ledit au moins un capteur de pression (6).

2. Système selon la revendication précédente, **caractérisé en ce qu'**il comprend deux capteurs de pression (6), chaque capteurs de pression (6) étant protégé par une membrane de protection (1) perméable au gaz et une pièce intermédiaire souple (8) formée d'un matériau élastiquement déformable étant agencée entre chaque capteur de pression (6) et la membrane de protection (1).

3. Système selon l'une des revendications précédentes, **caractérisé en ce que** la pièce intermédiaire souple (8) comprend un évidement interne amont (8b) situé du côté de la membrane de protection (1), et un logement interne aval (8c) situé du côté dudit au moins un capteur de pression (6), et l'évidement interne amont (8b) et le logement interne aval (8c) étant reliés fluidiquement l'un à l'autre par le passage interne (8a).

4. Système selon l'une des revendications précédentes, **caractérisé en ce que** la pièce intermédiaire souple (8) comprend un logement interne aval (8c) dans lequel est positionné ledit au moins un capteur de pression (6).

5. Système selon l'une des revendications précédentes, **caractérisé en ce que** la pièce intermédiaire souple (8) est formé d'un matériau polymère ou élastomère, de préférence un matériau de type élastomère thermoplastique TPE ou silicone.

6. Système selon l'une des revendications précédentes, **caractérisé en ce que** la pièce intermédiaire souple (8) comprend un rebord aval (12) délimitant le logement interne aval (8c), ledit rebord aval (12) venant prendre appui de manière étanche, autour dudit au moins un capteur de pression (6), sur la paroi (11) portant ledit au moins un capteur de pression (6) ou sur ledit au moins un capteur (6).

7. Système selon l'une des revendications précédentes, **caractérisé en ce que** la pièce intermédiaire souple (8) comprend une bordure amont (13) délimitant l'évidement interne amont (8b) et venant appuyer sur la membrane de protection (1).

8. Système selon l'une des revendications précédentes, **caractérisé en ce qu'**un boitier (7) comprenant un orifice d'entrée de gaz (7a) situé en regard de l'évidement amont (8b), permet de maintenir la pièce intermédiaire souple (8) au contact de la membrane de protection (1) et en position autour du capteur de pression (6).

9. Système selon l'une des revendications précédentes, **caractérisé en ce que** la pièce intermédiaire souple (8) comprend une paroi périphérique externe (8d) coopérant avec la paroi interne (7b) du boitier (7) de manière à assurer une étanchéité fluidique entre elles.

10. Système selon l'une des revendications précédentes, **caractérisé en ce que** le capteur de pression (6) est porté par une carte électronique (16) formant tout ou partie de la paroi (11) sur laquelle la pièce intermédiaire souple (8) vient prendre appui de manière étanche, via son rebord aval (12), autour dudit capteur de pression (6).

11. Système selon l'une des revendications précédentes, **caractérisé en ce que** la carte électronique (16) portant le ou les capteurs (6) est fixée dans le boitier (7) de manière à maintenir la membrane de protection (1) et la pièce intermédiaire souple (8) solidaires l'une de l'autre et assurer en outre une étanchéité entre la pièce intermédiaire souple (8) et la paroi (11) portant le capteur de pression (6).

12. Système selon l'une des revendications précédentes, **caractérisé en ce que** le boitier (7) comprend un premier embout (30) portant un orifice d'entrée de gaz et un second embout (31) portant un orifice de sortie de gaz, lesdits orifice d'entrée de gaz et orifice de sortie de gaz étant reliés par un passage de gaz interne (32), de préférence ledit passage de gaz interne (32) forme une partie du circuit de gaz (10).

13. Appareil de ventilation (20) de patient comprenant un circuit de gaz (10) apte à acheminer du gaz entre une source de gaz (23) et un patient, **caractérisé en ce qu'**il comporte en outre un système de mesure de pression de gaz (6, 16) selon l'une des revendications précédentes.

14. Appareil de ventilation selon la revendication 13, **caractérisé en ce que** le système de mesure de pression de gaz (6, 16) est agencé sur le circuit de gaz (10), de préférence au sein d'un boitier (7) venant se raccorder fluidique audit circuit de gaz (10) ou à une ligne de dérivation dudit circuit de gaz (10).
